# EUROPEAN PATENT SPECIFICATION

(11) **EP 3 565 534 B1**
(45) Date of publication and mention of the grant of the patent: **13.05.2020**
(21) Application number: 18708744.0
(22) Date of filing: 07.02.2018
(51) Int. Cl.: A61K 9/51, A61P 35/00

(54) **POLYMERIC NANOPARTICLES ENCAPSULATING A COMBINATION OF NATURAL BIO-ACTIVES TRANS-RESVERATROL(RSV) AND CELASTROL(CL), A PROCESS FOR THE PREPARATION AND USE THEREOF IN THE TREATMENT OF PROSTATE CANCER**
POLYMERE NANOPARTIKEL ZUR VERKAPSELUNG EINER KOMBINATION AUS NATÜRLICHEM BIOAKTIVEM TRANS-RESVERATROL (RSV) UND CELASTROL (CL), VERFAHREN ZUR HERSTELLUNG UND VERWENDUNG DAVON IN DER BEHANDLUNG VON PROSTATAKREBS
NANOPARTICULES POLYMÈRES ENCAPSULANT UNE COMBINAISON DES AGENTS BIO-ACTIFS NATURELS TRANS-RESVÉRATROL (RSV) ET CÉLASTROL (CL), LEUR PROCÉDÉ DE PRÉPARATION ET LEUR UTILISATION DANS LE TRAITEMENT DU CANCER DE LA PROSTATE

(30) Priority: 10.02.2017 IT 201700015178
(43) Date of publication of application: 13.11.2019
(73) Proprietor: Università degli Studi di Sassari, 07100 Sassari (IT)
(72) Inventor: SANNA, Vanna, 07100 Sassari (IT); SECHI, Mario, 07100 Sassari (IT); SIDDIQUI, Imtiaz Ahmad, Madison, WI 53706 (US); MUKHTAR, Hasan, Madison, WI 53706 (US)
(74) Representative: Metroconsult Srl
(86) International application number: PCT/IB2018/050757
(87) International publication number: WO 2018/146599

(56) References cited:
- WO-A1-2010/077642
- WO-A1-2014/134179
- VANNA SANNA ET AL: "Resveratrol-Loaded Nanoparticles Based on Poly(epsilin-caprolactone) and Poly(D,L-lactic-co-glycolic acid)-Poly(ethylene glycol) Blend for Prostate Cancer Treatment", MOLECULAR PHARMACEUTICS, vol. 10, no. 10, 7 October 2013 (2013-10-07), pages 3871-3881, XP055409831, US ISSN: 1543-8384, DOI: 10.1021/mp400342f
- NAN JI ET AL: "Effect of Celastrol on Growth Inhibition of Prostate Cancer Cells through the Regulation of hERG Channel In Vitro", BIOMED RESEARCH INTERNATIONAL, vol. 2015, 1 January 2015 (2015-01-01), pages 1-7, XP055410062, ISSN: 2314-6133, DOI: 10.1155/2015/308475
- YUAN LIU ET AL: "Synthesis, characterization and adsorption performance of molecularly imprinted nanoparticles for tripterine by precipitation polymerization", ANALYTICAL METHODS, vol. 6, no. 3, 1 January 2014 (2014-01-01) , pages 684-689, XP055409969, GBR ISSN: 1759-9660, DOI: 10.1039/C3AY41772G

## Description

### Technical Field

The present invention relates to polymeric nanoparticles for the combined administration of a number of substances of natural origin for the treatment of prostate cancer.

Specifically, the present invention relates to polymeric nanoparticles based on a blend of poly(ε-caprolactone) (PCL) and poly(D,L-lactic-co-glycolic acid)-poly(ethylene glycol) (PLGA-PEG) loaded with suitable amounts of resveratrol and celastrol. The invention also relates to the preparation of said polymeric nanoparticles loaded with resveratrol and celastrol and to their use for conveying said active substances in the treatment of prostate cancer.

### State of the Art

Although several naturally occurring substances have promising pharmacological activity against different types of cancers, their efficacy is often severely limited by a series of limitations due, for example, to their structural instability, poor solubility, non-efficient systemic release, poor bioavailability and/or intrinsic toxicity when administered as such. In an attempt to remedy these limitations, several studies have been directed to the identification of suitable systems, in particular nanosystems, for the most suitable delivery of these substances.

For example, nanoparticles based on different polymeric materials, such as PLGA (polylactic-co-glycolic acid), cellulose derivatives, dendrimers, cyclodextrins, have been studied to increase the cytotoxic activity of curcumin against prostate cancer cells (Thangapazham R.L. et al., Evaluation of a nanotechnology-based carrier for delivery of curcumin in prostate cancer cells. International Journal of Oncology. 2008;32(5):1119-1123).

Recently, Sanna and collaborators have proposed a polymeric nanosystem consisting of a blend of poly-caprolactone and PLGA-PEG, for the controlled release of resveratrol on several prostate cancer cell lines (Sanna V. et al., Resveratrol-loaded nanoparticles based on poly(ε-caprolactone) and poly(D,L-lactic-co-glycolic acid)-poly(ethylene glycol) blend for prostate cancer treatment. Mol. Pharm. 2013 Oct 7;10(10):3871-81).

Recently, Sanna and collaborators have proposed a polymeric nanosystem, based on poly-caprolactone, for the controlled release of celastrol on several prostate cancer cell lines (Sanna V. et al., Nanoencapsulation of natural triterpenoid celastrol for prostate cancer treatment. Int. J. Nanomedicine. 2015 Oct. 30;10:6835-6846).

Lately, among the emerging strategies for the treatment of cancers, a great interest has been focused on the combination of one or more synthetic chemotherapeutic agents with molecules of natural origin having a chemopreventive activity, with the aim of being able to ensure a better efficacy of treatments and a reduction of the side effects compared to the monotherapies used in conventional protocols. Furthermore, it is hoped that the possibility of combining different anticancer molecules and natural chemopreventive agents can offer the advantage of preventing the onset of the phenomenon of drug resistance by cancer cells, which represents a limitation of the currently used therapies. However, despite the interesting chemopreventive activities shown by different molecules of natural origin against prostate cancer, and the improvement of the effectiveness of the treatments of different types of cancers observed using a combination of synthetic molecules with natural molecules, their therapeutic application still remains rather limited.

To the present inventors' knowledge, to date the combination therapies studied to obtain an enhancement and/or in general an improvement of the anti-cancer activity mainly consist in the use of a natural molecule in association with synthetic chemotherapeutic agents.

On the contrary, no publications and/or patents are known describing the preparation of a single system of polymer nanoparticles loaded with at least two or more different molecules of natural origin, with antiproliferative/anti-cancer activity, in particular for the treatment (prevention and/or therapy) of prostate cancer.

### Technical Problem

Therefore, a need is strongly felt by the technicians of the field to have new systems for conveying and releasing (possibly in a controlled manner) a combination of several natural compounds, having a valid cytotoxic activity, in particular against prostate cancer cells, able to perform a chemopreventive/anti-cancer action and to obtain a significant enhancement and/or a general improvement in the activity of the bioactive molecules used, together with an improvement in the quality of life of cancer patients.

The object of the present invention is to provide an adequate response to the above technical problem.

### Summary of the Invention

The present inventors have now unexpectedly found that a suitable combination of at least two molecules of completely natural origin, one with a recognized chemopreventive activity and the other with a more marked cytotoxic action, incorporated/encapsulated in a suitable polymeric nanoparticle unit system, allows enhancing the anticancer activity of said combination with respect to that of the single molecules, both considered as such, and individually encapsulated in the nanosystem, and significantly improving the antiproliferative action on prostate cancer cell lines, with the potential to prevent and/or limit the induction of undesirable toxic and/or systemic effects, thus proving able to give an adequate response to the technical problem above highlighted.

An object of the present invention are therefore biocompatible polymeric nanoparticles (hereinafter **NP** as an acronym) based on a blend of poly(ε-caprolactone) (hereinafter, **PCL** as an acronym) and a poly(D,L-lactic-co-glycolic acid)-poly(ethylene glycol) conjugate (hereinafter, **PLGA-PEG-COOH** as an acronym) loaded with a combination of *trans*-resveratrol (hereinafter **RSV** as an acronym) and celastrol (hereinafter **CL** as an acronym), as set forth in the appended independent claim.

Another object of the present invention is a pharmaceutical composition comprising the above polymeric nanoparticles, as set forth in the appended independent claim.

Yet another object of the present invention is a method for the preparation of said polymeric nanoparticles referred to above, as set forth in the appended independent claim.

A further object of the present invention is the use of said polymeric nanoparticles referred to above for the treatment of prostate cancer, as set forth in the appended independent claim.

Preferred embodiments of the present invention are set forth in the appended dependent claims.

The preferred embodiments the present invention shown in the following description are described herein only by way of non-limiting example of the broad application scope of the invention, which will be apparent to the man skilled in the art.

### Brief Description of the Figures

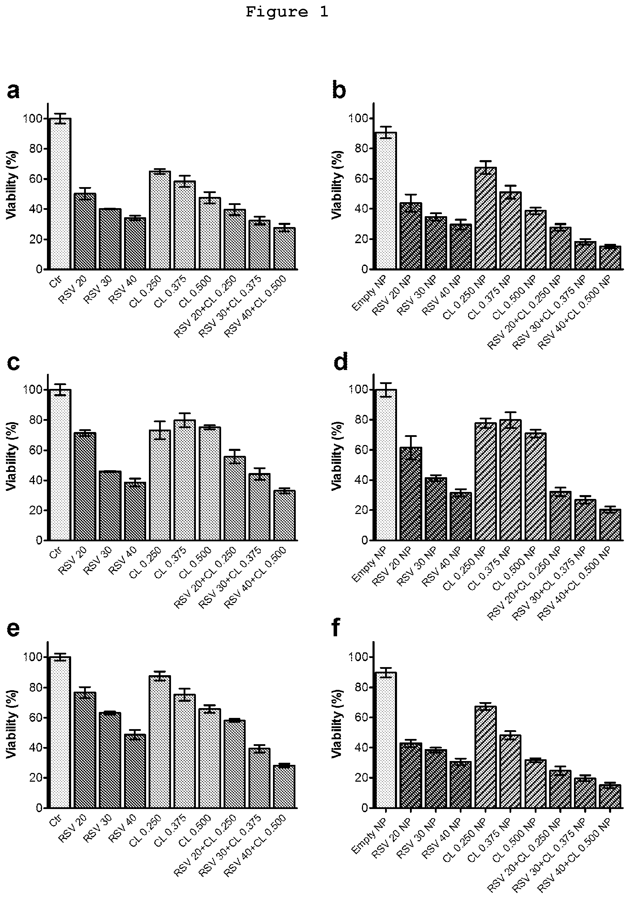

The present invention will be described hereinafter by way of example in no way limiting the scope of the same, by illustrating some preferred embodiments thereof. To this end, to better highlight the potential and peculiarities of the polymeric nanoparticles of the present invention, reference will also be made to the accompanying **Figure 1****.**

**Figure 1** shows the comparison between the viability of the tested prostate cancer cell lines (**C-42** cells (**a** & **b**); **DU-145** cells (**c** & **d**); and **PC-3** cells (**e** & **f**)) treated for 72 hours only with RSV at different doses (20, 30 and 40 µM); only with CL at different doses (0.250, 0.375 and 0.5 µM); with RSV + CL in combination at different doses (20 µM of RSV + 0,25 µM of CL, 30 µM of RSV + 0,375 µM of CL, and 40 µM of RSV + 0,5 µM of CL); and also treated with NP not loaded (empty), NP loaded with only RSV, NP loaded with only CL, and NP loaded with RSV + CL in combination with the same doses indicated above. (n = 3 or 5).

### Detailed Description of the Invention

### Foreword

Trans-resveratrol or *trans*-3,5,4'-trihydroxy-stilbene (**RSV**) is a non-flavonoid polyphenol mainly extracted from the root of *Polygonum Cuspidatum* (a perennial herbaceous plant belonging to the Polygonaceae family) and commonly also contained in some fruits like red grapes, blackberries, blueberries, raspberries and peanuts.

This phytoalexin, widely used in traditional Chinese medicine, produced by plants in response to climatic stress and in defence against pathogens such as bacteria or fungi, is well known for its anti-oxidant, anti-aging, anti-infective and protective effects at the cardiovascular and neurological level. Several *in vitro* and *in vivo* studies have shown the effectiveness of RSV as a preventive agent with a significant reduction in the formation and progression of prostate cancer.

Celastrol (**CL**), a triterpenoid compound extracted from the bark of the *Tripterygium wilfordii* plant root, widely used in traditional Chinese medicine, has shown interesting activities for the treatment of chronic inflammatory conditions, autoimmune diseases and neurodegenerative diseases. *In vitro* and *in vivo* studies have shown that treatment with CL results in the inhibition of the proteasome activity and the induction of apoptosis in PC-3 (androgen-receptors or AR-negative) and LNCaP (AR-positive) prostate cancer cells.

Unfortunately, the possible pharmacological use of RSV and CL, despite their interesting anticancer activities, is strongly limited due to the poor bioavailability of these molecules resulting, among other things, from their low solubility in water (the solubility of RSV is of 3 mg/100 mL, while CL is insoluble), chemical instability, which, for example, in the case of RSV, causes the conversion of the biologically active *trans* isomer into its inactive *cis* form, and a certain systemic toxicity, in particular of CL.

### Description

Therefore, are an object of the present invention biocompatible polymeric nanoparticles (NP) for the controlled co-release of the actives trans-resveratrol (RSV) and celastrol (CL), at least comprising:
- a biocompatible polymeric blend of poly(ε-caprolactone) (PCL) and poly(D,L-lactic-*co-*glycolic acid)-poly(ethylene glycol) conjugate co-polymer (PLGA-PEG-COOH); and
- an effective amount of a blend of trans-resveratrol (RSV) and celastrol (CL). Optionally, said NP further comprise an effective amount of at least one surfactant selected from the group consisting of: poloxamers, Pluronic® F-127, polysorbates, Tween® 80, polyvinyl alcohol, PVA; preferably, Pluronic® F-127 and PVA; more preferably Pluronic® F-127.

In the nanoparticles of the present invention, in said biocompatible polymeric blend, the two component polymers are present in a reciprocal mass ratio PCL:PLGA-PEG-COOH comprised from 3:0.5 to 0.5:2; preferably, 1.5:1.

In the nanoparticles of the present invention, in said blend of the two active principles (RVS and CL),
- RSV is present at a concentration of from 5 µM to 50 µM; preferably, from 10 µM to 45 µM; more preferably, from 20 µM to 40 µM. In preferred embodiments of the invention, said RSV concentration is 20 µM and/or 25 µM and/or 30 µM and/or 35 µM and/or 40 µM; and
- CL is present at a concentration from 0.050 µM to 5,000 µM; preferably, from 0.150 µM to 2.000 µM; more preferably, from 0.250 µM to 0.500 µM. In preferred embodiments of the invention, said concentration of CL is 0.250 µM and/or 0.325 µM and/or 0.375 µM and/or 0.425 µM and/or 0.500 µM.

The NPs of the present invention have a spherical shape and have an average hydrodynamic diameter (i.e., the diameter of the electrical dipole layer attached to the surface of the particle dispersed in a fluid) ranging from 50 nm to 350 nm; preferably, from 100 nm to 300 nm; more preferably, from 150 nm to 250 nm. In a preferred embodiment of the invention, said average hydrodynamic diameter is about 180-200 nm. In the nanoparticles of the present invention, said optional surfactant is present in aqueous solution in a concentration ranging from 0% to 2.0% by weight, with respect to the weight of the aqueous solvent; preferably from 0.05% to 1.0% by weight; more preferably, from 0.1% to 0.5% by weight. In a preferred embodiment of the invention, said surfactant is present in a concentration of 0.1%.

Another object of the present invention is a method for the preparation of the nanoparticles (NP) loaded with the above RSV and CL, wherein said method is a nanoprecipitation method at least comprising the following steps:
a) dissolving into a vessel, or into a reactor, provided with magnetic or mechanic stirring, according to the size of the vessel itself, the above polymeric blend of PCL:PLGA-PEG-COOH, RSV and CL into a suitable organic solvent, such as acetonitrile and/or acetone, preferably acetonitrile;
b) adding, under gentle stirring, the solution resulting from step a) into water, added or not with an effective amount of a surfactant, selected from the group consisting of poloxamers, Pluronic® F-127, polysorbates, Tween® 80, polyvinyl alcohol, PVA, preferably, Pluronic® F-127 and PVA, more preferably Pluronic® F-127, at a concentration of from 0% to 2.0%, preferably 0.1% by weight with respect to water, to give a colloidal, milky suspension of nanoparticles encapsulating the active principles;
c) keeping said suspension from step b) under stirring for about 1 hour at a temperature of from 15 to 30 °C, preferably at room temperature, to evaporate, remove, the organic solvent;
d) centrifuging the NPs from the suspension from step c), at 10,000 rpm for 5 minutes, and washing them with water several times, for example, three times, to remove the non-encapsulated actives (RSV and CL).

The method for the preparation of the NPs of the present invention, described above, has been carried out by substantially applying the same methodology and the same operating conditions described for *trans*-resveratrol (RSV) in the scientific paper by Sanna V., et al., Resveratrol-loaded nanoparticles based on poly(epsilon-caprolactone) and poly(D,L-lactic-co-glycolic acid)-poly(ethylene glycol) blend for prostate cancer treatment. Mol. Pharm. 2013 Oct 7;10(10):3871-81, which, as a result, is included herein as a reference in its entirety.

The NPs obtained as described above were characterized in terms of morphology, particle size, zeta potential, encapsulation efficiency, chemical-physical and thermal analysis, and *in vitro* release studies, applying the same methodologies and operating conditions described in Sanna V., et al., Resveratrol-loaded nanoparticles based on poly(epsilon-caprolactone) and poly(D,L-lactic-co-glycolic acid)-poly(ethylene glycol) blend for prostate cancer treatment. Mol. Pharm. 2013 Oct 7;10(10):3871-81, which, as a result, is included herein as a reference in its entirety.

In particular, it was found, as previously noted, that the NPs of the invention are spherical in shape, characterized by an average hydrodynamic diameter in the range from 50 nm to 350 nm; more preferably, from 150 nm to 250 nm; even more preferably, from 180 nm to 200 nm.

Moreover, the production yield of the method was high, of around 68-77%, while the encapsulation efficiency of the active principles was even higher, around 78-98%.

Furthermore, the NPs of the present invention have been proved capable of controlling the *in vitro* release of the encapsulated actives (RSV and CL). In fact, at pH 7.4 (in physiological conditions), RSV and CL showed very different release rates; while RSV was released almost completely after the first 4 hours of the test, CL was characterized by a very prolonged release, with only about 12% released within the first 6 hours, about 50% after 24 hours, and 75% approximately after 48 hours.

Finally, *in vitro* cytotoxicity studies were performed in order to evaluate whether co-administration of encapsulated RSV and CL would have allowed an increase in the antiproliferative efficacy compared to single molecules, both as such and formulated in nanosystems, on androgen-dependent (**C-42** cells) and androgen-independent (**DU-145** and **PC-3** cells) prostate cancer cell lines (PCa).

The antiproliferative efficacy of the NPs of the present invention was evaluated using the MTT assay (3-[4,5-dimethylthiazol-2-yl]-2,5-diphenyl tetrazolium bromide) applying the procedure described in Sanna V., et al., Resveratrol-loaded nanoparticles based on poly(epsilon-caprolactone) and poly(D,L-lactic-co-glycolic acid)-poly(ethylene glycol) blend for prostate cancer treatment. Mol. Pharm. 2013 Oct 7;10(10):3871-81, which, as a result, is included herein as a reference in its entirety.

As shown in **Figure 1**, the tested prostate cancer cell lines (in the order, C-42, DU-145, and PC-3 cells) were treated for 72 hours, respectively, only with RSV; only with CL; with RSV + CL in combination; with NP not loaded with actives, with NP loaded with only RSV, with NP loaded with only CL, and with NP loaded with RSV + CL in combination. Each experiment was repeated 3 or 5 times (n = 3 or 5).

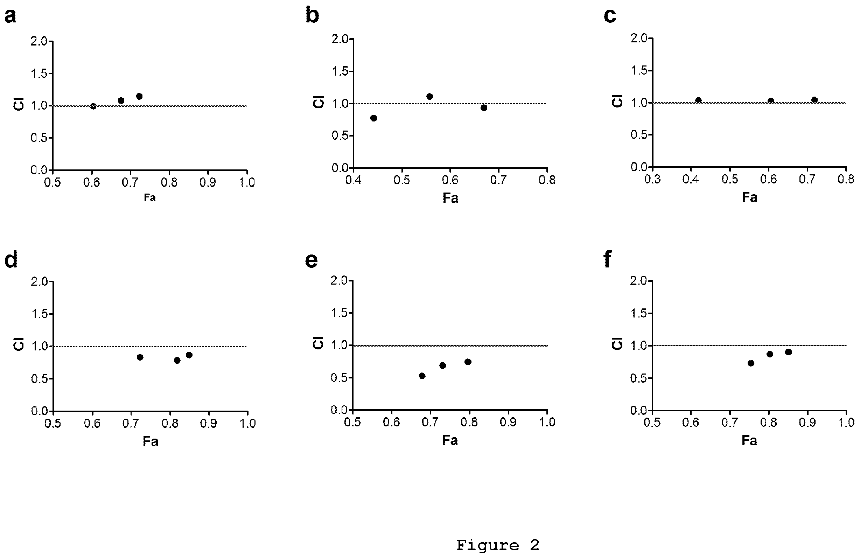

As can be seen from the graph, in all cancer cell lines the treatment with non-encapsulated RSV and CL alone produces a reduction of viability that is generally dose-dependent (**Figure 1**: a, c, e); the treatment with the combination of non-encapsulated RSV + CL produces an increase in cytotoxicity, compared to treatment with RSV or CL alone, due to an additive effect, as can be seen from the combination values (CI) (**Figure 2****:** a, b , c), resulted, in general, between 0.9 and 1.10 (calculated according to the so-called *"Loewe additivity"* method).

The encapsulation of RSV or CL in NP (Figures b, d, f) generally leads to an increase in cytotoxicity compared to the profiles observed for the free molecules.

Also the NPs loaded with the combination of RSV + CL all show an antiproliferative activity that is significantly higher than both the non-encapsulated combination and the single encapsulated molecules. In this case, this increase in cytotoxicity is due to a synergistic effect, as shown by the values of CI (**Figure 2**: d, e, f), which are between 0.5 and 0.9, recorded for the three cell lines.

Comparison of the cell viability data after treatment with the non-encapsulated RSV + CL combination and, respectively, encapsulated in the NPs shows an increase in cytotoxicity which also reaches about 60%.

Specifically, on C-42 androgen-dependent PCa cell lines, the NPs loaded with the RSV + CL combination, at the three tested combination doses, show a cytotoxicity of about 30%, 40 % and 45% respectively higher than at the corresponding doses of the non-encapsulated RSV + CL combination. In turn, on the cell lines of the androgen-independent PCa, the antiproliferative activity of NPs loaded with the RSV + CL combination is improved/increased on average by about 40% on the DU-145 cells, and of values between 45% and 60% on PC-3 cells, compared to the non-encapsulated RSV + CL combination.

This clearly shows that the co-administration of RSV + CL encapsulated in the NPs of the present invention causes a significant and unexpected enhancement of the anti-cancer effects, compared to those of the single molecules (encapsulated or not) and of their non-encapsulated combination, attributable to a synergistic effect.

### Experimental Section

The following experimental section describes some of the characteristic aspects of the present invention, without however limiting the wide potential for use thereof.

### Materials and methods

For the preparation of the NPs of the present invention, the necessary materials were substantially found on the market, similarly to what is indicated in Sanna V., et al., Resveratrol-loaded nanoparticles based on poly(epsilon-caprolactone) and poly(D,L-lactic-co-glycolic acid)-poly(ethylene glycol) blend for prostate cancer treatment. Mol. Pharm. 2013 Oct 7;10(10):3871-81; and in Sanna V. et al., Nanoencapsulation of natural triterpenoid celastrol for prostate cancer treatment. Int. J. Nanomedicine. 2015 Oct. 30;10:6835-6846, which are incorporated herein as a reference.

The PLGA-PEG-COOH conjugated co-polymer was instead prepared as indicated in the following Example 1.

### Example 1: Preparation of the PLGA-PEG-COOH conjugate

To a solution of PLGA-COOH (3.0 g, 0.166 mmol: lactide/glycolide ratio 50:50, terminal -COOH, viscosity 0,20 dL/g) [Purac Biomaterials, Gorinchem, Nl] in methylene chloride (10 mL), were added N-hydroxyisuccinimide (NHS, 76 mg, 0.66 mmol) (Sigma-Aldrich, Steinheim, DE) and 1-ethyl-3-(3-dimethylaminopropyl)-carbodiimide (EDC, 140 mg, 0.72 mmol) [Sigma-Aldrich], and the reaction mixture was magnetically stirred at room temperature for 12 hours under a nitrogen atmosphere. PLGA-NHS was obtained by precipitation with cold diethyl ether (10 mL) as a white solid, which was filtered and washed repeatedly with a cold mixture of diethyl ether and methanol (in a 1:1 ratio by volume, v:v), and then dried under vacuum and in a stream of nitrogen (yield, about 95%). The PLGA-NHS activated polymer (3.0 g, 0.166 mmol) was dissolved in anhydrous chloroform (10 mL), and the NH₂-PEG-COOH compounds (0.75 g, 0.22 mmol: MW = 3.400) [JenChem Technology, USA) and N,N-diisopropylethylamine (DIPEA, 42 mg, 0.75 mmol) [Sigma-Aldrich] were then added under magnetic stirring. The reaction mixture was kept under magnetic stirring for 24 hours. The conjugated co-polymer was obtained by treating the mixture with previously cooled diethyl ether (yield, about 90%). The resulting PGLA-PEG-COOH was dried under vacuum, characterized by ¹H NMR (500 MHz: Brucker Avance 500) and used for the preparation of the NPs without further treatment.
¹H NMR (500 MHz, CDCl₃): δ 5.23(m, -OC-CH(CH₃)O-, PLGA), 4.78(m, -OC-CH₂O-, PLGA), 3.65(s, -CH₂CH₂O-, PEG), 1.56(brs, -OCCHCH₃O-, PLGA)

### Example 2: Preparation of the nanoparticles of the invention

PCL [Sigma-Aldrich] (56.4 mg) and PLGA-PEG-COOH [from Example 1] (37.6 mg), in a mass ratio of 1.5:1, RSV [Sigma-Aldrich] (6 mg), and CL [Baoji Guokang BioTechnology Co., Ltd, Republic of China] (0.12 mg) were dissolved in acetonitrile (6 mL) and, under gentle stirring, added dropwise in water (20 mL) containing Pluronic® F-127 [Sigma-Aldrich] (0.1% w/w) to give a milky colloidal suspension containing the NPs of the invention. Said suspension was made to evaporate under stirring at room temperature to remove the organic solvent; subsequently, the NPs were centrifuged at 10,000 rpm for 5 min. and washed with water (at least 3 times) to remove the non-encapsulated actives. The NPs loaded with the actives were used immediately for subsequent tests, or lyophilized and stored at -50 °C.

The NPs without actives and the NPs loaded with the single actives, to be used as a comparison, were prepared in the same way.

The yield of the particle preparation reaction was about 74%, while the encapsulation efficiency of the active principles was about 92%.

### Advantages/Industrial Applicability of the Invention

While, as noted above, the potential pharmacological use of RSV and CL, despite their interesting antiproliferative/cytotoxic activities, is strongly limited because of their poor bioavailability, the co-encapsulation of said molecules in the NPs of the invention, described above and in the appended claims, has provided the advantage of increasing its solubility and chemical stability, reducing its potential systemic toxicity, controlling its release and improving its overall bioavailability. Moreover, the above polymeric nanoparticles loaded with the natural active principles RSV and CL have proved to be very effective/active against the cancer lines of prostate cancer, thus being unexpectedly and advantageously promising for use in the treatment of said pathology.

A further object of the present invention is therefore the use of the above polymeric nanoparticles loaded with the RSV and CL actives for the delivery and controlled release of said actives to the desired site of a patient's organism.

Therefore, a further object of the present invention is the use of the above polymeric nanoparticles loaded with the RSV and CL actives for the preparation of a pharmaceutical composition for the treatment of prostate cancer.

Therefore, a further object of the present invention is a pharmaceutical composition comprising the above polymeric nanoparticles loaded with the RSV and CL actives for use in the treatment of prostate cancer.

## Claims

1. Biocompatible polymeric nanoparticles (NP) for the controlled co-release of the natural actives *trans*-resveratrol (RSV) and celastrol (CL), at least comprising:
- a biocompatible polymeric blend of poly(ε-caprolactone) (PCL) and poly(D,L-lactic-co-glycolic acid)-poly(ethylene glycol) conjugate co-polymer (PLGA-PEG-COOH); and
- an effective amount of a blend of *trans*-resveratrol (RSV) and celastrol (CL).

2. The NPs according to claim 1, further comprising or not an effective amount of a surfactant.

3. The NPs according to claim 1 or 2, wherein in said polymeric blend the two component polymers are present in a reciprocal mass ratio PCL:PLGA-PEG-COOH comprised from 3:0.5 to 0.5:1.

4. The NPs according to any one of the preceding claims, wherein, in said blend of the two active principles,
- RSV is present at a concentration from 5 µM to 50 µM; and
- CL is present at a concentration from 0.050 µM to 5.000 µM.

5. The NPs according to any one of the preceding claims, wherein said surfactant is selected from the group consisting of poloxamers, Pluronic® F-127, polysorbates, Tween® 80, polyvinyl alcohol, PVA; said surfactant being present in aqueous solution at a concentration from 0% to 2.0% by weight, relative to the aqueous solvent.

6. The NPs according to any one of the preceding claims, **characterized by** a spherical shape and an average hydrodynamic diameter from 50 nm to 350 nm.

7. A method for preparing the nanoparticles (NPs) according to any one of claims 1 to 6, wherein said method is a nanoprecipitation method at least comprising the following steps:
a) dissolving into a vessel provided with magnetic or mechanic stirring said polymeric blend of PCL:PLGA-PEG-COOH, RSV and CL into an organic solvent;
b) adding, under gentle stirring, the solution resulting from step a) into water, added or not with an effective amount of a surfactant, to give a colloidal, milky suspension of nanoparticles encapsulating the active principles;
c) keeping said suspension from step b) under stirring at a temperature from 15 to 30°C to evaporate, remove, the organic solvent;
d) centrifuging the NPs from the suspension from step c), and washing them with water to remove the non-encapsulated actives (RSV and CL).

8. A method according to claim 7, wherein
- in said step a), said solvent is acetonitrile and/or acetone;
- in said step b), said surfactant is selected from the group consisting of poloxamers, Pluronic® F-127, polysorbates, Tween® 80, polyvinyl alcohol, PVA; said surfactant being present in aqueous solution at a concentration from 0% to 2.0% by weight relative to the aqueous solvent;
- in said step d), the NPs are centrifuged at 10,000 rpm for 5 min; and
wherein the global yield of the method is approximately 68-77% and the encapsulation of the active principles is approximately 78-98%.

9. Use of the NPs according to any one of claims 1 to 6 for *in vitro* treatment of the C-42 and/or DU-145 and/or PC-3 prostate cancer cell lines.

10. A pharmaceutical composition comprising the nanoparticles according to any one of claims 1 to 6 for use in the treatment of prostate cancer.

## Patentansprüche

1. Biokompatible polymere Nanopartikel (NP) für die kontrollierte Co-Freisetzung der natürlichen Wirkstoffe trans-Resveratrol (RSV) und Celastrol (CL), mindestens umfassend:
- eine biokompatible Polymermischung aus Poly(ε-Caprolacton) (PCL) und Poly(D,L-Milch-Co-Glycolsäure)-Poly(ethylenglycol)-Konjugat-Copolymer (PLGA-PEG-COOH); und
- eine wirksame Menge einer Mischung aus *trans-Resveratrol* (RSV) und Celastrol (CL).

2. Die NP nach Anspruch 1, die außerdem eine wirksame Menge eines Tensids enthalten oder nicht.

3. Die NP nach Anspruch 1 oder 2, wobei in der genannten Polymermischung die Zweikomponenten-Polymere in einem reziproken Massenverhältnis PCL:PLGA-PEG-COOH umfassend 3:0,5 bis 0,5:1 vorhanden sind.

4. Die NP nach einem der vorhergehenden Ansprüche, wobei in der genannten Mischung der beiden Wirkstoffe
- RSV in einer Konzentration von 5 µM bis 50 µM vorhanden ist; und
- CL in einer Konzentration von 0,050 µM bis 5,000 µM vorhanden ist.

5. Die NP nach einem der vorhergehenden Ansprüche, wobei besagtes Tensid aus der Gruppe ausgewählt ist, bestehend aus Poloxameren, Pluronic® F-127, Polysorbaten, Tween® 80, Polyvinylalkohol, PVA; wobei besagtes Tensid in wässriger Lösung in einer Konzentration von 0% bis 2,0 Gew.-%, bezogen auf das wässrige Lösungsmittel, vorliegt.

6. Die NP nach einem der vorhergehenden Ansprüche, **gekennzeichnet durch** eine Kugelform und einen durchschnittlichen hydrodynamischen Durchmesser von 50 nm bis 350 nm.

7. Verfahren zur Herstellung der Nanopartikel (NP) nach einem der Ansprüche 1 bis 6, wobei das Verfahren ein Nanopräzipitationsverfahren ist, das mindestens die folgenden Schritte umfasst:
a) Lösen der besagten Polymermischung aus PCL:PLGA-PEG-COOH, RSV und CL in einem organischen Lösungsmittel in einem mit magnetischer oder mechanischer Rührvorrichtung ausgestatteten Gefäß;
b) Zugabe, unter leichtem Rühren, der aus Schritt a) resultierenden Lösung zu Wasser, mit oder ohne Zugabe einer wirksamen Menge eines Tensids, um eine kolloidale, milchige Suspension von Nanopartikeln zu erhalten, die die Wirkstoffe einkapseln;
c) Halten der Suspension aus Schritt b) unter Rühren bei einer Temperatur von 15 bis 30°C, um das organische Lösungsmittel zu verdampfen, zu entfernen;
d) Zentrifugieren der NP aus der Suspension aus Schritt c) und Waschen derselben mit Wasser, um die nicht eingekapselten Wirkstoffe (RSV und CL) zu entfernen.

8. Verfahren nach Anspruch 7, wobei
- in Schritt a) das genannte Lösungsmittel Acetonitril und/oder Aceton ist;
- in besagtem Schritt b) das besagte Tensid aus der Gruppe ausgewählt ist, bestehend aus Poloxameren, Pluronic® F-127, Polysorbaten, Tween® 80, Polyvinylalkohol, PVA; wobei das besagte Tensid in wässriger Lösung in einer Konzentration von 0 bis 2,0 Gew.-%, bezogen auf das wässrige Lösungsmittel, vorhanden ist;
- in besagtem Schritt d) die NP bei 10.000 U/min für 5 Minuten zentrifugiert werden; und
wobei die globale Ausbeute des Verfahrens etwa 68-77% und die Einkapselung der Wirkstoffe etwa 78-98% beträgt.

9. Verwendung der NP nach einem der Ansprüche 1 bis 6 zur *in vitro-*Behandlung der Prostatakrebs-Zelllinien C-42 und/oder DU-145 und/oder PC-3.

10. Pharmazeutische Zusammensetzung umfassend die Nanopartikel nach einem der Ansprüche 1 bis 6 zur Verwendung bei der Behandlung von Prostatakrebs.

## Revendications

1. Nanoparticules polymères biocompatibles (NP) pour la co-libération contrôlée des actifs naturels *trans-*resvératrol (RSV) et célastrol (CL), comprenant au moins :
- un mélange polymère biocompatible de poly(ε-caprolactone) (PCL) et de copolymère conjugé poly(acide D,L-lactique-co-glycolique)-poly(éthylène glycol) (PLGA-PEG-COOH) ; et
- une quantité efficace d'un mélange de trans-resvératrol (RSV) et de célastrol (CL).

2. NP selon la revendication 1, comprenant ou non en outre une quantité efficace d'un tensio-actif.

3. NP selon l'une des revendications 1 ou 2, dans lesquelles, dans ledit mélange polymère, les deux polymères constitutifs sont présents dans un rapport massique réciproque PCL : PLGA-PEG-COOH allant de 3 : 0,5 à 0,5 : 1.

4. NP selon l'une quelconque des revendications précédentes, dans lesquelles, dans ledit mélange des deux principes actifs,
- le RSV est présent à une concentration de 5 µM à 50 µM ; et
- le CL est présent à une concentration de 0,050 µM à 5,000 µM.

5. NP selon l'une quelconque des revendications précédentes, dans lesquelles ledit tensio-actif est choisi dans le groupe consistant en les poloxamères, le Pluronic® F-127, les polysorbates, le Tween® 80, l'alcool polyvinylique, PVA, ledit tensio-actif étant présent en solution aqueuse à une concentration de 0 % à 2,0 % en poids, par rapport au solvant aqueux.

6. NP selon l'une quelconque des revendications précédentes, **caractérisées par** une forme sphérique et un diamètre hydrodynamique moyen de 50 nm à 350 nm.

7. Procédé de préparation des nanoparticules (NP) selon l'une quelconque des revendications 1 à 6, dans lequel ledit procédé est un procédé de nanoprécipitation comprenant au moins les étapes suivantes :
a) dissoudre dans un récipient muni d'une agitation magnétique ou mécanique ledit mélange polymère de PCL : PLGA-PEG-COOH, le RSV et le CL dans un solvant organique ;
b) ajouter, sous agitation douce, la solution résultant de l'étape a) dans de l'eau, additionnée ou non d'une quantité efficace d'un tensio-actif, pour donner une suspension laiteuse, colloïdale, de nanoparticules encapsulant les principes actifs ;
c) maintenir ladite suspension provenant de l'étape b) sous agitation à une température de 15 à 30°C pour faire évaporer, retirer le solvant organique ;
d) centrifuger les NP à partir de la suspension provenant de l'étape c) et les laver avec de l'eau pour retirer les actifs non encapsulés (RSV et CL).

8. Procédé selon la revendication 7, dans lequel :
- dans ladite étape a), ledit solvant est l'acétonitrile et / ou l'acétone ;
- dans ladite étape b), ledit tensio-actif est choisi dans le groupe consistant en les poloxamères, le Pluronic® F-127, les polysorbates, le Tween® 80, l'alcool polyvinylique, PVA, ledit tensio-actif étant présent en solution aqueuse à une concentration de 0 % à 2,0 % en poids par rapport au solvant aqueux ;
- dans ladite étape d), les NP sont centrifugés à 10 000 tpm pendant 5 min ; et
dans lequel le rendement global du procédé est approximativement de 68 - 77 % et l'encapsulation des principes actifs est d'approximativement 78 - 98 %.

9. Utilisation des NP selon l'une quelconque des revendications 1 à 6 pour le traitement *in vitro* des lignées cellulaires du cancer de la prostate C-42 et / ou DU-145 et / ou PC-3.

10. Composition pharmaceutique comprenant les nanoparticules selon l'une quelconque des revendications 1 à 6 pour une utilisation dans le traitement du cancer de la prostate.
